# EUROPEAN PATENT APPLICATION

(11) **EP 2 742 868 A1**
(43) Date of publication of application: **18.06.2014**
(21) Application number: 13188449.6
(22) Date of filing: 14.10.2013
(51) Int. Cl.: A61B 8/00, A61B 8/08

(54) **Ultrasound apparatus and method of inputting information into same**

(30) Priority: 12.12.2012 KR 20120144659
(71) Applicant: Samsung Medison Co., Ltd., Gangwon-do 250-870 (KR)
(72) Inventor: Lee, Yun-hee, Gangwon-do (KR); Lee, Seung-ju, Gangwon-do (KR); Kim, Yong-soo, Gangwon-do (KR); Kim, Tae-hun, Gangwon-do (KR)
(74) Representative: Lorenz, Markus

(57) **Abstract**

In an ultrasound apparatus comprising a user input unit for receiving a touch input, provided are a method and apparatus for receiving an input from a user for setting a gain value, determining a coordinate matching mode between the user input unit and an output unit, and adjusting a gain value of ultrasound data based on the user input and the coordinate matching mode. In addition, provided are a method and apparatus for applying a compensation value for ultrasound data, through a user input.

## Description

### CROSS-REFERENCE TO RELATED PATENT APPLICATION

This application claims the benefit of Korean Patent Application No. 10-2012-0144659, filed on December 12, 2012, in the Korean Intellectual Property Office, the disclosure of which is incorporated herein in its entirety by reference.

### BACKGROUND OF THE INVENTION

### 1. Field of the Invention

The present invention relates to an ultrasound apparatus for receiving a touch input, and a method of inputting information into the ultrasound apparatus.

### 2. Description of the Related Art

An ultrasound apparatus emits an ultrasound signal (generally, 20 KHz frequency or higher) onto a predetermined part of an object by using a probe and acquires an image of the predetermined part inside the object by using information regarding a reflected echo signal. In particular, the ultrasound apparatus is used for medical purposes, such as foreign substance detection and injury measurement and observation inside an object. The ultrasound signal has advantages of being more stable than X-rays, displayable in real-time, and safe without radiation exposure, and thus, the ultrasound apparatus is widely used together with other image diagnosis devices, such as an X-ray diagnosis device, a computed tomography (CT) device, a magnetic resonance imaging (MRI) device, and a nuclear medicine diagnosis device.

In general, an amplitude or strength of an ultrasound signal passing through tissue inside an object decreases along a transfer distance. Attenuation of an ultrasound signal is shown in a form wherein an amplitude of the ultrasound signal decreases as a distance along which the ultrasound signal passes increases. A strength of an attenuated and received ultrasound echo signal may not be constant. That is, an ultrasound image based on an ultrasound echo signal may not have uniform brightness or may have bad quality in a partial region. Therefore, a system by which a user can easily compensate for for the attenuation of a signal when generating an ultrasound image is required.

### SUMMARY OF THE INVENTION

According to an aspect of the present invention, there is provided a method of inputting information, the method including: displaying an ultrasound image based on ultrasound data acquired from an object; receiving, from a user, through a first region included in a user input unit, a first gain line for setting a gain value to be applied to the ultrasound data; determining a coordinate matching mode between a second region included in a screen on which the ultrasound image is displayed and the first region; and adjusting a gain value of the ultrasound data based on the coordinate matching mode and the first gain line.

The gain value of the ultrasound data may include at least one of a time gain compensation (TGC) value, a lateral gain compensation (LGC) value, an overall gain value, and a partial gain value.

The receiving may include: detecting a user input of tapping and/or dragging one or more locations in the first region; and acquiring a gain value corresponding to the user input.

The coordinate matching mode may be a relative mode in which a vertical axis coordinate of the first region matches a depth axis coordinate of the second region or an absolute mode in which a horizontal axis coordinate and the vertical axis coordinate of the first region match a horizontal axis coordinate and the depth axis coordinate of the second region.

When the coordinate matching mode is the relative mode, the horizontal axis coordinate of the first region from which the user input starts may match a predetermined point on a horizontal axis of the second region.

The predetermined point may be located on a second gain line corresponding to the gain value of the ultrasound data before the adjusting.

When the coordinate matching mode is the absolute mode, coordinates of the first region from which the user input starts may be coordinates of the second region matching coordinates of the first region.

The coordinate matching mode may be determined in advance by the user.

The user input unit may include at least one of a touch screen, a touch panel, a touch pad, and a track ball.

The method may further include displaying on a screen an ultrasound image based on the ultrasound data having the adjusted gain value.

The method may further include displaying on a screen at least one of a second gain line corresponding to the gain value of the ultrasound data before the adjusting and a third gain line corresponding to the adjusted gain value.

The acquiring of the gain value may include acquiring a gain value corresponding to one location in the first region when the user input is a one-point input of tapping and/or dragging the one location.

The acquiring of the gain value may include acquiring a gain value corresponding to a center of two locations in the first region when the user input is a two-point input of tapping and/or dragging the two locations.

The acquiring of the gain value may include acquiring a gain value corresponding to a boundary in a direction in which three or more locations in the first region are dragged when the user input is a three-point input of tapping and/or dragging the three or more locations.

The acquiring of the gain value may include acquiring a gain value corresponding to a location maintained from a one-point input from among two locations of a two-point input when the user input is a multi-input of changing from a one-point input of tapping and/or dragging one location in the first region to a two-point input of tapping and/or dragging the two locations in the first region.

The acquiring of the gain value may include acquiring a gain value corresponding to one location in the first region when the user input is a one-point input of tapping and/or dragging the one location in the first region and when a detection signal is input through a third region included in the user input unit.

According to another aspect of the present invention, there is provided an apparatus including: an acquisition unit for acquiring ultrasound data from an object; an output unit for displaying an ultrasound image based on the ultrasound data; a user input unit, which includes a first region and receives, from a user through the first region, a first gain line for setting a gain value to be applied to the ultrasound data; a mode checking unit for determining a coordinate matching mode between a second region included in the output unit and the first region; and an image processing unit for adjusting a gain value of the ultrasound data based on the coordinate matching mode and the first gain line.

According to another aspect of the present invention, there is provided a method of inputting information, the method including: displaying an ultrasound image on a screen based on ultrasound data acquired from an object; receiving, from a user, through a first region included in a user input unit, a compensation location to which a compensation value for the ultrasound data is to be applied; and applying the compensation value to the ultrasound data based on a vertical axis of the first region, which matches a depth axis of the ultrasound image, and the compensation location.

The compensation value may include at least one of brightness, chroma, color, and definition of the ultrasound image.

According to another aspect of the present invention, there is provided a non-transitory computer-readable storage medium having stored therein program instructions, which when executed by a computer, perform the method of inputting information.

### BRIEF DESCRIPTION OF THE DRAWINGS

The above and other features and advantages of the present invention will become more apparent by describing in detail exemplary embodiments thereof with reference to the attached drawings in which:
FIG. 1 is a perspective view of an ultrasound system for diagnosing an object through an ultrasound signal;
FIG. 2 is a block diagram of an ultrasound apparatus according to an embodiment of the present invention;
FIG. 3 is a flowchart illustrating a method of inputting ultrasound information, according to an embodiment of the present invention;
FIGS. 4A to 4C illustrate examples of receiving a user input according to embodiments of the present invention;
FIG. 5 illustrates a relative mode according to an embodiment of the present invention;
FIG. 6 illustrates an absolute mode according to an embodiment of the present invention;
FIGS. 7A and 7B illustrate displaying a gain line according to an embodiment of the present invention;
FIG. 8 illustrates a one-point input according to an embodiment of the present invention;
FIG. 9 illustrates a one-point input according to another embodiment of the present invention;
FIGS. 10A to 10C illustrate a two-point input according to an embodiment of the present invention;
FIG. 11 illustrates a three-point input according to an embodiment of the present invention;
FIG. 12 illustrates a three-point input according to another embodiment of the present invention;
FIG. 13 illustrates a multi-input according to an embodiment of the present invention;
FIG. 14 illustrates a one-point input, which is input together with a detection signal, according to an embodiment of the present invention;
FIGS. 15A and 15B illustrate using a track ball according to embodiments of the present invention;
FIG. 16 is a flowchart illustrating a method of inputting ultrasound information, according to another embodiment of the present invention;
FIG. 17 is a flowchart illustrating a method of inputting ultrasound information, according to another embodiment of the present invention; and
FIGS. 18A to 18C illustrate an automatic compensation process according to embodiments of the present invention.

### DETAILED DESCRIPTION OF THE INVENTION

Although general terms as currently widely used as possible are selected as the terms used in the present invention while taking functions in the present invention into account, they may vary according to an intention of one of ordinary skill in the art, judicial precedents, or the appearance of new technology. In addition, in specific cases, terms intentionally selected by the applicant may be used, and in this case, the meaning of the terms will be disclosed in a corresponding description of the invention. Accordingly, the terms used in the present invention should be defined not by simple names of the terms but by the meaning of the terms and the content over the present invention.

In the specification, when a certain part "includes" a certain component, this indicates that the part may further include another component instead of excluding another component unless there is a disclosure stating otherwise. In addition, the term, such as "...unit" or "...module," which is disclosed in the specification, indicates a unit for processing at least one function or operation, and this may be implemented by hardware, software, or a combination thereof.

As used herein, the term "and/or" includes any and all combinations of one or more of the associated listed items. Expressions such as "at least one of," when preceding a list of elements, modify the entire list of elements and do not modify the individual elements of the list.

In the specification, "ultrasound image" indicates an image of an object, which is acquired using an ultrasound signal. The object may indicate a part of the human body. For example, the object may include an organ, such as the liver, the heart, the nuchal translucency (NT), the brain, the breast, the abdomen, or the like, a fetus, or the like.

The ultrasound image may be variously realized. For example, the ultrasound image may be at least one of an amplitude mode (A-mode) image, a brightness mode (B-mode) image, a color mode (C-mode) image, and a Doppler mode (D-mode) image. In addition, according to an embodiment of the present invention, the ultrasound image may be a two-dimensional image or a three-dimensional image.

In the specification, "user" may be a medical expert, such as a medical practitioner, a nurse, a medical laboratory technologist, a sonographer, or the like, but is not limited thereto.

Hereinafter, embodiments of the present invention will be described in detail with reference to the accompanying drawings so that one of ordinary skill in the art may easily realize the present invention. However, the present invention may be embodied in many different forms and should not be construed as being limited to the embodiments set forth herein. In the drawings, parts irrelevant to the description are omitted to clearly describe the present invention, and like reference numerals denote like elements throughout the specification.

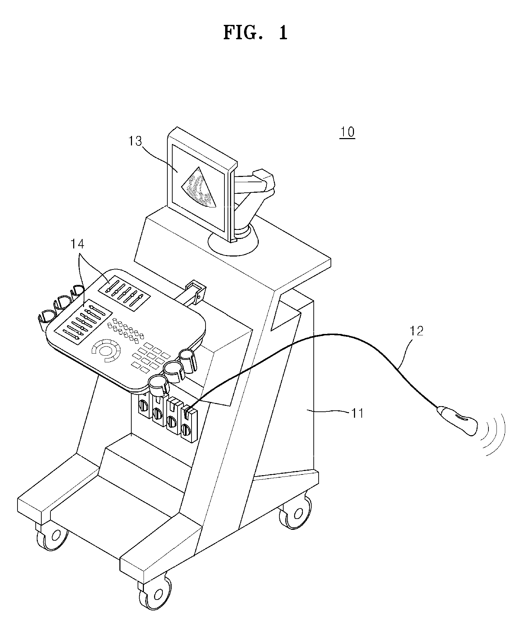

FIG. 1 is a perspective view of an ultrasound system 10 for diagnosing an object through an ultrasound signal. The ultrasound system 10 according to an embodiment of the present invention includes a main body 11, one or more probes 12, a display unit 13, and a control panel.

A user positions the probe 12, which transmits an ultrasound signal, to be close to an object and acquires ultrasound data based on an echo signal received from the object. Thereafter, the user may diagnose the object through an ultrasound image generated by analyzing the ultrasound data and displayed on the display unit 13. The control panel according to an embodiment of the present invention may include one or more gain adjustment panels 14 for adjusting a gain value of the ultrasound data.

When the object is diagnosed through the ultrasound system 10, the user adjusts brightness of a desired part of an image by conceptually connecting the gain adjustment panel 14 that is hardware and a depth axis (generally, a vertical axis) of the ultrasound image of the display unit 13. For the user to naturally adjust the brightness of the desired part of the image, one or more slide bars included in the gain adjustment panel 14 should be simultaneously moved to the left and the right. Thus, a method and apparatus for easily and naturally adjusting a gain value of the ultrasound data while the user maintains visual focus on the display unit 13 are required.

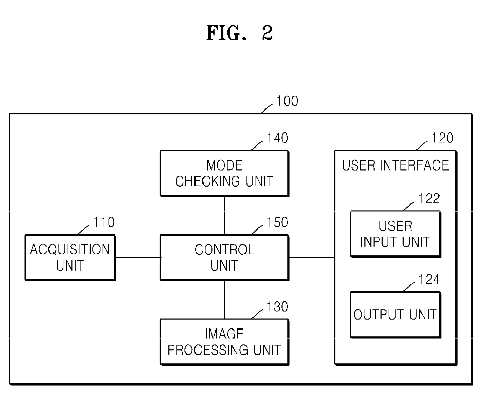

FIG. 2 is a block diagram of an ultrasound apparatus 100 according to an embodiment of the present invention. The ultrasound apparatus 100 may include an acquisition unit 110, a user interface 120, an image processing unit 130, a mode checking unit 140, and a control unit 150. The ultrasound apparatus 100 may further include other general-use components in addition to the components shown in FIG. 2.

The ultrasound apparatus 100 is a device capable of acquiring ultrasound data from an object by using ultrasound waves and providing a graphic user interface (GUI) for setting a gain value of the ultrasound data to a user.

The ultrasound apparatus 100 can be realized in various forms. For example, the ultrasound apparatus 100 described in the specification may be realized in a form of not only a stationary terminal but also a mobile terminal. Examples of the mobile terminal are a PACS viewer, a laptop computer, a tablet PC, and the like.

Components included in the ultrasound apparatus 100 will now be described one by one.

The acquisition unit 110 acquires ultrasound data of an object. The ultrasound data according to an embodiment of the present invention may be two-dimensional or three-dimensional ultrasound data of the object. In addition, the ultrasound data may include Doppler data indicating a motion of the object.

According to an embodiment of the present invention, the acquisition unit 110 may include a probe (not shown) for transmitting and receiving an ultrasound signal and a beamformer (not shown) for performing transmission focusing and reception focusing of the ultrasound signal. The probe according to an embodiment of the present invention may include at least one of one-dimensional (1 D), 1.5D, 2D (matrix), and 3D probes.

The acquisition unit 110 may not only directly acquire the ultrasound data by scanning the object as described above but may also acquire the ultrasound data acquired in advance by using another device to scan the object or by accessing an image acquired by an external device and stored in the external server.

That is, the acquisition unit 110 may receive the ultrasound data in a wired or wireless manner by using one or more components capable of communicating between the ultrasound apparatus 100 and an external device. For example, the acquisition unit 110 may acquire the ultrasound data by using a short distance communication module, a mobile communication module, a wireless Internet module, a wired Internet module, or the like.

The short distance communication module indicates a module for short distance communication. For short distance communication technology, a wireless LAN (Wi-Fi), Bluetooth, Bluetooth low energy (BLE), ultra wideband (UWB), ZigBee, near field communication (NFC), Wi-Fi Direct (WFD), infrared data association (IrDA), or the like may be used.

The mobile communication module transmits and receives a wireless signal to and from at least one of a base station, an external terminal, and a server in a mobile communication network. The wireless Internet module indicates a module for a wireless Internet access and may be embedded in the acquisition unit 110 or be external. The wired Internet module indicates a module for a wired Internet access.

According to an embodiment of the present invention, the acquisition unit 110 may receive the ultrasound data from an external device through wired or wireless communication. The external device according to an embodiment of the present invention may include a portable phone, a smart phone, a laptop computer, a tablet PC, an e-book terminal, a digital broadcast terminal, a personal digital assistant (PDA), a portable multimedia player (PMP), a digital camera, or the like, but is not limited thereto.

The acquisition unit 110 may acquire the ultrasound data in a wired or wireless manner from not only the external device but also a hospital server or a cloud server via a picture archiving and communication system (PACS).

The user interface 120 includes an input means and an output means for interacting between the ultrasound apparatus 100 and the user. For example, the user interface 120 may include a user input unit 122 for receiving an input from the user and an output unit 124 for providing information from the ultrasound apparatus 100 to the user.

The user input unit 122 refers to a means for inputting, by the user, data for controlling the ultrasound apparatus 100 into the ultrasound apparatus 100. The user input unit 122 may receive various types of control inputs, e.g., a touch input, from the user.

The user input unit 122 may include a key pad, a track ball, a mouse, a dome switch, a touch pad (a capacitive overlay touch pad, a resistive overlay touch pad, an Infrared beam touch pad, a surface acoustic wave touch pad, an integral strain gauge touch pad, a piezoelectric touch pad, or the like), a touch panel, a jog wheel, a jog switch, or the like, but is not limited thereto. In particular, the user input unit 122 may also include a touch screen having a layer structure of the touch pad and the output unit 124 to be described below.

The touch screen may detect not only a real touch but also a proximity touch. In the specification, the real touch refers to when a pointer actually touches a screen, and the proximity touch indicates that the pointer does not actually touch a screen but approaches the screen within a predetermined distance. In the specification, the pointer refers to a tool for touching or proximity-touching a specific part of a displayed screen. Examples of the pointer are a stylus pen, a finger, and the like.

Although not shown, various sensors may be prepared inside or around the touch screen to detect a touch or a proximity touch on the touch screen. An example of the sensors for detecting a touch on the touch screen is a tactile sensor. The tactile sensor indicates a sensor that detects a contact of a specific object and is as sensitive or more sensitive in comparison to a human touch. The tactile sensor may detect various kinds of information, such as roughness of a contact surface, solidity of a contact object, temperature of a contact point, and the like.

Another example of the sensors for detecting a touch on the touch screen is a proximity sensor. The proximity sensor indicates a sensor for detecting the presence/absence of an object approaching a predetermined detection surface or an object existing nearby, without a mechanical contact and by using a force of an electromagnetic field or an infrared ray. Examples of the proximity sensor are a transmissive photoelectric sensor, a direct reflective photoelectric sensor, a mirror reflective photoelectric sensor, a high-frequency oscillation type proximity sensor, a capacitive proximity sensor, a magnetic type proximity sensor, an infrared proximity sensor, and the like.

The user input unit 122 may receive various types of touch inputs from the user as described above. A user input detected by the user input unit 122 may include a tap, a touch & hold, a double-tap, a drag, panning, a flick, a drag & drop, a swipe, or the like, according to a touch pattern. In addition, the user input may include not only a one-point input, a two-point input, and a three-point input according to the number of detected inputs but also a multi-input in another form, which includes a combination of the one-point input, the two-point input, and the three-point input. Each user input will be described below with reference to detailed embodiments.

The output unit 124 may display information processed by the ultrasound apparatus 100. For example, the output unit 124 may display an ultrasound image of the object on a screen and may also display a user interface (UI) or a graphic user interface (GUI) related to function setting.

The output unit 124 may include at least one of a liquid crystal display, a thin film transistor-liquid crystal display, an organic light-emitting diode, a flexible display, a 3D display, and an electrophoretic display. The ultrasound apparatus 100 may include two or more output units 124 according to an implementation form of the ultrasound apparatus 100.

When the output unit 124 and the user input unit 122 are layered in a touch screen, the output unit 124 may also be used as an input device in addition to an output device.

The image processing unit 130 processes the ultrasound data by using various methods. That is, the image processing unit 130 may generate an ultrasound image from the ultrasound data or adjust a gain value of the ultrasound data. In other words, the image processing unit 130 may adjust the ultrasound data according to a user input and generate an ultrasound image to be displayed on the output unit 124 based on the adjusted data.

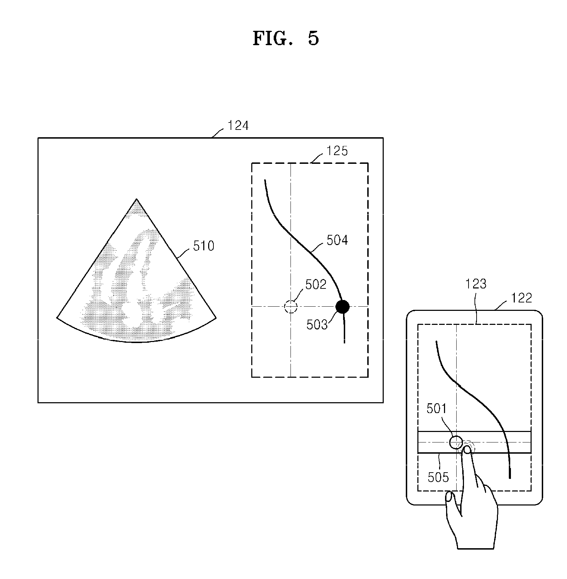

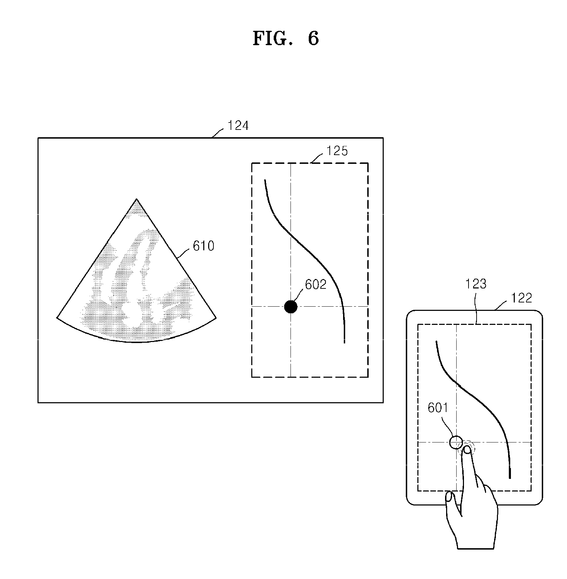

The mode checking unit 140 determines a coordinate matching mode between the user input unit 122 and the output unit 124. That is, coordinates of a partial region of the user input unit 122 and a partial region of the output unit 124 may match each other, and this will be described in detail with reference to FIGS. 5 and 6. The coordinate matching mode may be a relative mode in which a vertical axis coordinate of a first region that is a partial region of the user input unit 122 matches a depth axis coordinate of a second region that is a partial region of the output unit 124 or an absolute mode in which a horizontal axis coordinate and the vertical axis coordinate of the first region match a horizontal axis coordinate and the depth axis coordinate of the second region.

That is, the mode checking unit 140 determines a coordinate matching mode determined by a selection made by the user or a selection made by an internal system of the ultrasound apparatus 100 as the relative mode or the absolute mode. The coordinate matching mode determined by the mode checking unit 140 may be used to adjust a gain value of the ultrasound data in the image processing unit 130 together with a user input.

The mode checking unit 140 may acquire a predetermined value of the coordinate matching mode from a storage unit (not shown). That is, although not shown, the ultrasound apparatus 100 may further include the storage unit, which may store information and programs for controlling the ultrasound apparatus 100 or store input/output data (e.g., a preset gain value, an ultrasound image, object information, probe information, application information, a body marker, and the like).

The control unit 150 controls a general operation of the ultrasound apparatus 100. That is, the control unit 150 may generally control the acquisition unit 110, the user input unit 122, the output unit 124, the image processing unit 130, the mode checking unit 140 , and so forth.

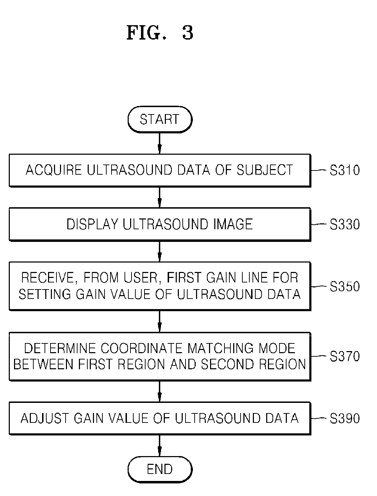

A method of inputting ultrasound information through a touch input by using components included in the ultrasound apparatus 100 will now be described with reference to FIG. 3. FIG. 3 is a flowchart illustrating a method according to an embodiment of the present invention. The flowchart of FIG. 3 includes sequential operations processed by acquisition unit 110, the user interface 120, the image processing unit 130, the mode checking unit 140, and the control unit 150 of the ultrasound apparatus 100 of FIG. 2. Thus, although omitted hereinafter, the description related to the components of FIG. 2 is also applied to the flowchart of FIG. 3.

In operation S310, the ultrasound apparatus 100 acquires ultrasound data of an object. That is, the ultrasound apparatus 100 may transmit an ultrasound signal to the object and generate ultrasound image data based on an echo signal received from the object. Alternatively, the ultrasound apparatus 100 may receive ultrasound data from an external device or server in a wired or wireless manner.

In operation S330, the ultrasound apparatus 100 displays an ultrasound image. That is, the ultrasound apparatus 100 may generate an ultrasound image by processing the ultrasound data acquired in operation S310 and display the generated ultrasound image on a screen.

In operation S350, the ultrasound apparatus 100 receives a first gain line, from a user, for setting a gain value of the ultrasound data. That is, the ultrasound apparatus 100 may detect, through the user input unit 122, a touch input of the user for forming the first gain line and acquire a gain value corresponding to a location of the detected touch input.

As described above, the gain value may include at least one of a TGC value, an LGC value, an overall gain value, and a partial gain value. The TGC value is used to compensate for a decrease in a magnitude of an ultrasound signal as it travels along a depth of the object. The LGC value is used to compensate for the differences in attenuation of different ultrasound beams due to differences in their respective transfer paths. The overall gain value and the partial gain value indicate gain values that are compensated for with respect to the overall ultrasound data and a partial ultrasound data, respectively. Hereinafter, for convenience of description, the TGC value is described as an example of the gain value.

The first gain line is detected through the user input unit 122, such as a touch panel, a touch screen, or the like, and may indicate a touch input of the user for adjusting a gain value of the ultrasound data. That is, the first gain line may not indicate a physically shaped line displayed on the user input unit 122 or the output unit 124 but may indicate information regarding a touch input of the user. The first gain line according to an embodiment of the present invention may be detected through the first region that is a partial region included in the user input unit 122.

A user input of forming the first gain line may include various types of tap and/or drag inputs. The term "tap" indicates an action of the user touching a screen using a finger or a touch tool (e.g., an electronic pen) and immediately lifting the finger or the touch tool from the screen without moving the finger or the touch tool to another location before discontinuing the touch. The term "drag" indicates an action of the user touching a screen using a finger or a touch tool and moving the finger or the touch tool to another location on the screen while maintaining the touch. The various types of user input will be described below in detail with reference to FIGS. 5 to 15.

In operation S370, the ultrasound apparatus 100 determines a coordinate matching mode. That is, the ultrasound apparatus 100 may determine a coordinate matching mode between the first region that is a partial region of the user input unit 122 and the second region that is a partial region included in the output unit 124, which were described above in detail. The ultrasound apparatus 100 may determine the coordinate matching mode between the two regions based on an input for determining the coordinate matching mode, which is received from the user, or by checking a coordinate matching mode stored in advance in the storage unit.

In operation S390, the ultrasound apparatus 100 adjusts the gain value of the ultrasound data. That is, the ultrasound apparatus 100 may adjust the gain value of the ultrasound data based on a gain line acquired from the first gain line in operation S350 and the coordinate matching mode determined in operation S370.

In addition, in operation S390, the ultrasound apparatus 100 may display an ultrasound image based on the ultrasound data of which the gain value has been adjusted. That is, the user may observe in real time an ultrasound image to which an adjusted gain value is applied while adjusting the gain value through the user input unit 122. As the user sets the gain value to be higher, an ultrasound image may be brighter, and as the user sets the gain value to be lower, an ultrasound image may be darker.

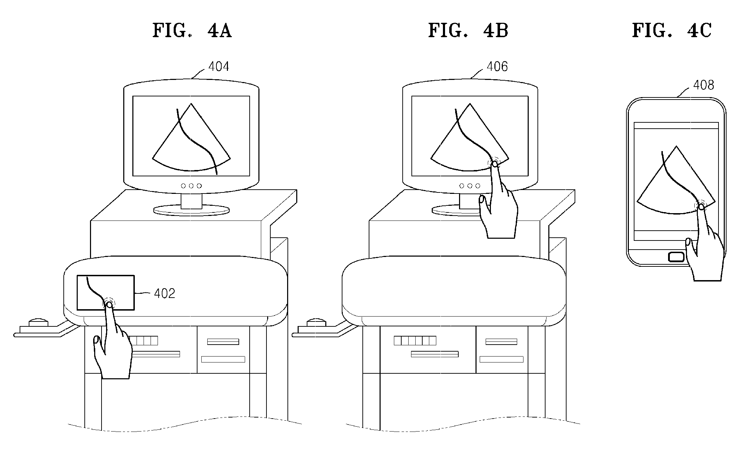

FIGS. 4A to 4C illustrate examples of receiving a user input according to embodiments of the present invention.

As shown in FIG. 4A, the ultrasound apparatus 100 may receive a touch input of a user through a user input unit 402 located in a control panel separately from an output unit 404 for displaying an ultrasound image.

As shown in FIG. 4B, when an output unit 406 for displaying an ultrasound image is a touch screen, the ultrasound apparatus 100 may receive a touch input of the user through the output unit 406. That is, the output unit 406 may also act as a user input unit.

As shown in FIG. 4C, the ultrasound apparatus 100 may be realized by a mobile terminal. For example, the ultrasound apparatus 100 may be realized by various types of mobile terminals, such as a PAVS viewer, a portable phone, a smart phone, a laptop computer, a tablet PC, and the like. An output unit 408 of the ultrasound apparatus 100 shown in FIG. 4C may also act as a user input unit similarly to the embodiment shown in FIG. 4B. That is, the output unit 408 of the ultrasound apparatus 100 may be an input means for detecting a touch input of the user.

Embodiments of receiving a user input in the ultrasound apparatus 100 are not limited to the embodiments of FIGS. 4A to 4C. That is, the ultrasound apparatus 100 may display an ultrasound image and receive a user input by using various methods.

Before describing an embodiment of adjusting a gain value in the ultrasound apparatus 100, the coordinate matching mode and the gain line will now be first described. Although a case where a gain value and a gain line are for TGC is shown and described as an example with reference to FIGS. 5 to 7, the present invention is not limited thereto, a case of LGC may also be applicable as well as the case of TGC.

FIG. 5 illustrates the relative mode according to an embodiment of the present invention. The relative mode indicates a mode in which a vertical axis coordinate of a first region 123 that is a partial region of the user input unit 122 matches a depth axis coordinate of a second region 125 that is a partial region of the output unit 124. A horizontal axis coordinate of the first region 123 matches a location on a second gain line 504 corresponding to a gain value of an ultrasound image 510 displayed on the output unit 124.

That is, when a user selects a location 501 in the first region 123 through a touch input in the relative mode, a location 503 on the second gain line 504 is selected instead of a location 502 in the second region 125, which matches coordinates of the location 501 in the first region 123. In other words, even though the user selects any location in a region 505 having the same vertical axis coordinate in the first region 123, the location 503 on the second gain line 504 in the second region 125 is selected.

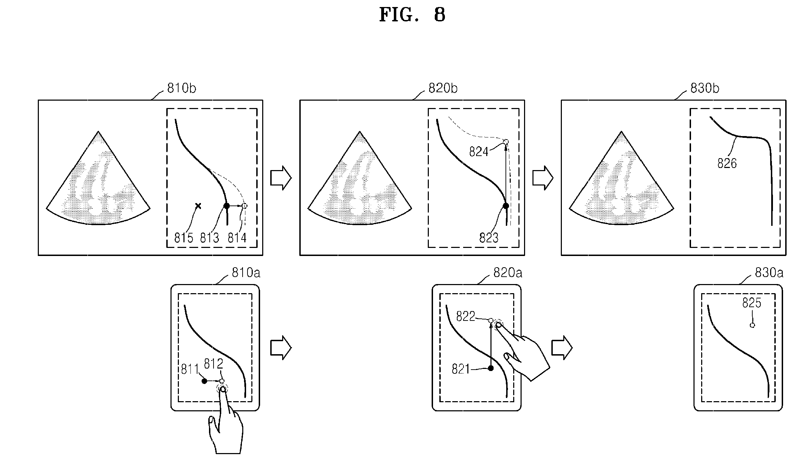

Since the coordinate matching mode is independent to a type of a user input, a user input is not limited to a user input using one finger as shown in FIG. 5. Various user inputs will be described below with reference to FIGS. 8 to 14.

FIG. 6 illustrates the absolute mode according to an embodiment of the present invention. The absolute mode indicates a mode in which a horizontal axis coordinate and a vertical axis coordinate of the first region 123 match a horizontal axis coordinate and a depth axis coordinate of the second region 125. That is, coordinates between the first region 123 and the second region 125 absolutely match each other.

When a user selects a location 601 in the first region 123 through a touch input in the absolute mode, a location 602 in the second region 125, which matches the location 601, is selected. That is, the location 602 in the second region 125 is selected based on the horizontal-axis vertical-axis coordinates of the location 601 in the first region 123.

In the embodiments of FIGS. 5 and 6, the ultrasound apparatus 100 may determine a detection region and a non-detection region to prevent a case where a touch input unintended by the user is detected.

In detail with reference to FIG. 6, the detection region may indicate a region having a predetermined size, which includes the location 601 at which a touch input of the user starts. For example, when a point in the first region 123 at which two long-short-long lines cross is the location 601, the ultrasound apparatus 100 may determine a circle region of a solid line, which includes the location 601, as the detection region.

The non-detection region may be a region except for the detection region in the first region 123 of the user input unit 122, and the ultrasound apparatus 100 may receive a touch input only for the detection region. That is, the ultrasound apparatus 100 may not detect a touch input in the non-detection region. Accordingly, even though an unnecessary and unintended touch by the user is detected in the first region 123, the ultrasound apparatus 100 may not recognize a touch input in the non-detection region.

When a user input of the user tapping and/or dragging one or more locations in the first region 123 ends, the ultrasound apparatus 100 may clear the detection region and the non-detection region after a predetermined time has elapsed. That is, the ultrasound apparatus 100 may maintain the detection region and the non-detection region for the predetermined time even though the user input ends.

The ultrasound apparatus 100 may selectively determine the detection region and the non-detection region by a user input or system settings. That is, in a case of a multi-input in which various types of user input are used, the ultrasound apparatus 100 may selectively determine the detection region and the non-detection region by the user's input or system settings.

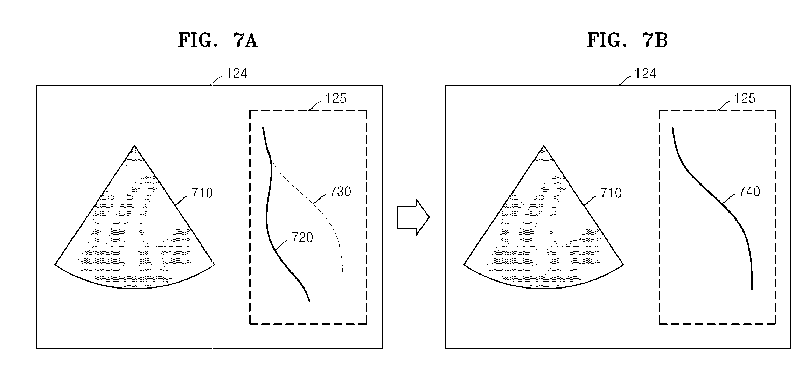

FIGS. 7A and 7B illustrate displaying a gain line according to an embodiment of the present invention. As described above, the first gain line indicates a gain line formed by a user on a user input unit. In FIGS. 7A and 7B, an embodiment in which the ultrasound apparatus 100 displays at least one of a second gain line and a third gain line is shown and described.

The second gain line is a gain line corresponding to ultrasound data and an ultrasound image before a gain value thereof is adjusted. That is, the second gain line indicates a gain line before a gain value is adjusted by a user input. The third gain line indicates a gain line after a gain value is adjusted by a user input.

According to the embodiment of FIG. 7A, the ultrasound apparatus 100 may display at least one of a second gain line 720 and a third gain line 730 in the second region 125 of the output unit 124 together with an ultrasound image 710.

That is, when a user input for changing a gain value of ultrasound data of the ultrasound image 710 is received from the user, the ultrasound apparatus 100 may display at least one of the second gain line 720 before the gain value is changed and the third gain line 730 after the gain value is changed. According to an embodiment of the present invention, the ultrasound apparatus 100 may display the second gain line 720 and the third gain line 730 with different chromas, colors, brightnesses, or the like to visually distinguish the second gain line 720 and the third gain line 730 from each other or may display any one of the second gain line 720 and the third gain line 730 with a dashed line or a long-short-long line to identify a shape thereof.

Although FIG. 7A shows that the ultrasound apparatus 100 displays the third gain line 730 with a dashed line, the second gain line 720 may be instead displayed with a dashed line.

In FIG. 7B, the ultrasound apparatus 100 may display only a third gain line 740 indicating a changed gain value when a touch input for forming the first gain line, which is detected from the user, ends. That is, since a gain value before a change does not have to be displayed because a touch input of the user is no longer detected, the ultrasound apparatus 100 may display only the third gain line 740.

Unlike FIGS. 7A and 7B, the ultrasound apparatus 100 may not display the second gain line and the third gain line on a screen. That is, the ultrasound apparatus 100 may not display information itself regarding a gain value and may be used only for detecting a user input and internally adjusting a gain value of ultrasound data therein.

When the ultrasound apparatus 100 does not display a gain line, the ultrasound apparatus 100 may display a change of a gain value by adjusting luminance of the ultrasound image 710 displayed on the output unit 124. That is, when the user increases a gain value of the ultrasound image 710, the ultrasound apparatus 100 may display the ultrasound image 710 by increasing luminance of a location at which the gain value is changed, i.e., making the location brighter.

Embodiments of adjusting a gain value based on the first gain line and the coordinate matching mode in the ultrasound apparatus 100 will now be described in detail. Although it is shown hereinafter for convenience of description that an output unit and a user input unit are separately implemented, the output unit and the user input unit may be implemented in various forms as described above with reference to FIG. 4. In addition, although a TGC value is illustrated in FIGS. 8 to 17, the embodiments are not limited thereto, and the embodiments of adjusting a gain value may also be applied to an LGC value.

FIG. 8 illustrates a one-point input according to an embodiment of the present invention. The one-point input indicates that a user input detected by the ultrasound apparatus 100 is an input of tapping and/or dragging one location on a user input unit 810a, 820a, or 830a. FIG. 8 shows a one-point input when the coordinate matching mode is the relative mode.

A solid line shown on the user input units 810a, 820a, and 830a is for comparison with output units 810b, 820b, and 830b and is irrelevant to operations of the user input units 810a, 820a, and 830a.

First, a user touches a location 811 of a user input unit 810a with a finger and drags the finger to a location 812 while maintaining the touch input. Since the coordinate matching mode is the relative mode, the location 811 matches a location 813 on a second gain line displayed as a solid line on an output unit 810b (in the absolute mode, the location 811 matches a location 815).

According to detection of a one-point input of dragging the finger from the location 811 to the location 812, the ultrasound apparatus 100 moves the second gain line from the location 813 to a location 814. That is, the ultrasound apparatus 100 moves the second gain line from the location 813 in a second region to the location 814 by a distance matching a distance between the location 811 and the location 812 in a first region. According to the movement of the second gain line from the location 813 to the location 814, a gain value of ultrasound data increases as much as the moved distance. The ultrasound apparatus 100 may display the increased gain value by displaying the second gain line moved to meet the location 814.

Thereafter, the user touches a location 821 with the finger and drags the finger to a location 822 while maintaining the touch input. Likewise, in the relative mode, the location 821 matches a location 823 on the second gain line displayed as a solid line on an output unit 820b. According to a one-point input of dragging the finger from the location 821 to the location 822, the ultrasound apparatus 100 may move the second gain line to a location 824 and display the second gain line as a dashed line. A distance by which the second gain line has moved in a depth axis direction may match a distance that the finger moved during the one-point input, which is detected by a user input unit 820a.

According to the embodiment described above, the ultrasound apparatus 100 may adjust a gain value based on the one-point input of the user and simultaneously change a luminance value of an ultrasound image based on the adjusted gain value. That is, the ultrasound apparatus 100 may adjust the brightness of the ultrasound image by reflecting a gain value adjusted according to a user input.

Finally, when the user ends the touch input at a location 825 of a user input unit 830a, the ultrasound apparatus 100 displays a third gain line 826 passing through a location on an output unit 830b, which corresponds to the location 825. That is, the ultrasound apparatus 100 adjusts a gain value to correspond to the location 825 and displays the third gain line 826 corresponding to the adjusted gain value.

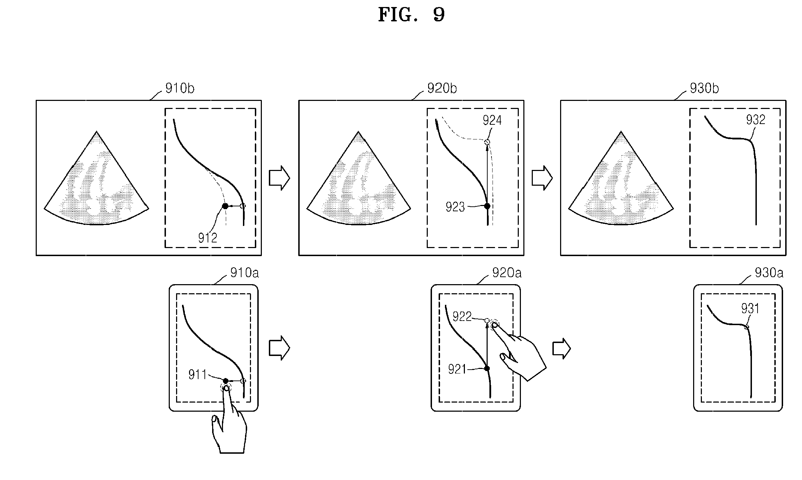

FIG. 9 illustrates a one-point input according to another embodiment of the present invention. Unlike FIG. 8, FIG. 9 illustrates a case where the coordinate matching mode is the absolute mode.

In the absolute mode, a user selects a location corresponding to a second gain line displayed as a solid line on an output unit 910b with a finger and drags the finger to a location 911. Accordingly, the ultrasound apparatus 100 may move the second gain line to a location 912 on the output unit 910b, which corresponds to the location 911.

Alternatively, when the user directly touches the location 911 instead of a location on a solid line displayed on a user input unit 910a, the ultrasound apparatus 100 may directly adjust a gain value to a gain value at a location 912 that corresponds to the location 911. That is, the ultrasound apparatus 100 may display gain values so that the gain values change, in a discontinuous manner instead of a continuous manner, to the location 912 from the second gain line displayed as a solid line.

Thereafter, when a user input unit 920a detects a one-point input of dragging the finger from a location 921 to a location 922, locations 923 and 924 respectively corresponding to the locations 921 and 922 are selected. That is, the ultrasound apparatus 100 may determine the locations 923 and 924 in a second region shown as a dashed rectangle on an output unit 920b based on coordinates of the locations 921 and 922 and a distance between the locations 921 and 922 in a first region shown as a dashed line on the user input unit 920a.

That is, according to a user input of dragging the finger from the location 921 to the location 922, the ultrasound apparatus 100 may adjust a gain value of ultrasound data so that the gain value of the ultrasound data corresponds to a shape of a third gain line displayed as a dashed line on the output unit 920b.

Finally, when the user ends the touch input at a location 931, the ultrasound apparatus 100 may adjust a gain value to correspond to a shape of a third gain line 932 and display the third gain line 932 on a screen.

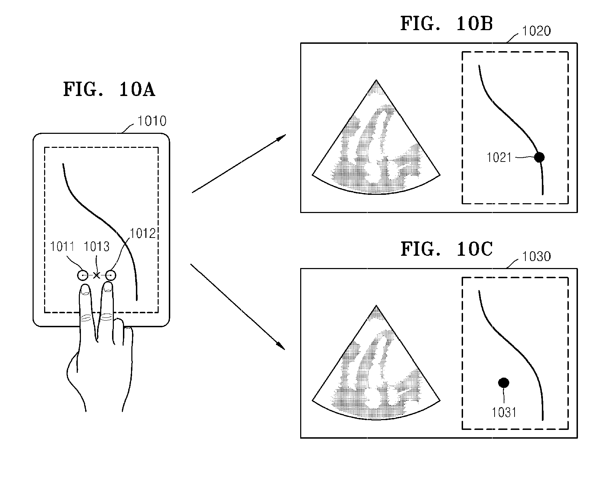

FIGS. 10A to 10C illustrate a two-point input according to an embodiment of the present invention. The two-point input indicates a user input of tapping and/or dragging two locations on a user input unit 1010 with two fingers.

According to an embodiment of the present invention, when a two-point input is received, the ultrasound apparatus 100 may adjust ultrasound data by acquiring a location of the center of two detected locations and acquiring a gain value corresponding to the location of the center.

That is, as shown in FIG. 10A, when a user input of touching a location 1011 and a location 1012 with two fingers, the ultrasound apparatus 100 may determine the user input similarly to a one-point input of selecting a location 1013 that is the center of the locations 1011 and 1012.

FIG. 10B shows a case where the two-point input is detected in the relative mode. That is, when the location 1013 is selected according to the detection of the user input at the locations 1011 and 1012, the ultrasound apparatus 100 selects a location 1021, which is a location on a depth axis corresponding to the location 1013, on a second gain line displayed as a solid line.

Thereafter, when a user drags the two fingers while touching the locations 1011 and 1012, the ultrasound apparatus 100 may determine a location corresponding to the center of two moved locations and acquire a gain value at the determined location. The ultrasound apparatus 100 may display an ultrasound image by adjusting a gain value of the ultrasound data to correspond to the user input and adjusting a luminance value of the ultrasound image according to the adjusted gain value.

FIG. 10C shows a case where the two-point input is detected in the absolute mode. That is, when the location 1013 is selected, the ultrasound apparatus 100 may determine a location 1031 according to a matching relationship between a first region displayed as a dashed line on the user input unit 1010 and a second region displayed as a dashed line on an output unit 1030.

The ultrasound apparatus 100 may display an ultrasound image by acquiring a gain value corresponding to the location 1031 and adjusting the acquired gain value. The ultrasound apparatus 100 may display a third gain line, which is a new gain line corresponding to the adjusted gain value, on a screen.

When the user of the ultrasound apparatus 100 adjusts a gain value by using a hardware device, using two fingers such as a forefinger and a middle finger as in a two-point input is typical. Thus, according to the embodiment of FIGS. 10A to 10C, the user may familiarly and efficiently adjust a gain value by directly adjusting the gain value using two fingers.

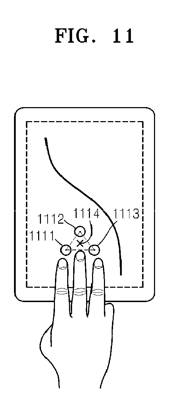

FIG. 11 illustrates a three-point input according to an embodiment of the present invention. The three-point input indicates a user input of tapping and/or dragging three or more locations on a user input unit.

As shown in FIG. 11, when a three-point input of selecting three locations 1111, 1112, and 1113 is detected, the ultrasound apparatus 100 may select the center of the three locations 1111, 1112, and 1113 similarly to the selection according to the two-point input described with reference to FIG. 10A. That is, the ultrasound apparatus 100 may select a location 1114 of the center of the three locations 1111, 1112, and 1113 and acquire a gain value corresponding to the location 1114.

Since a case where a three-point input is detected in the absolute mode or the relative mode is similar to the description made with reference to FIGS. 10B and 10C, a detailed description thereof is omitted. Although FIG. 11 shows that the user selects the three locations 1111, 1112, and 1113, the three-point input is not limited thereto and includes cases where four or five locations are selected.

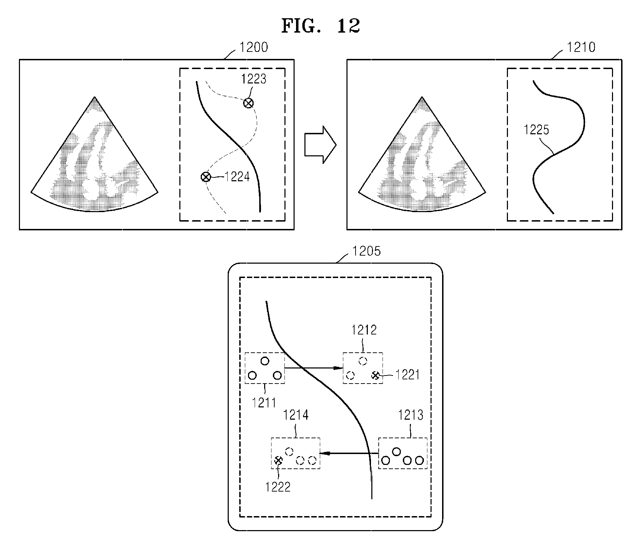

FIG. 12 illustrates a three-point input according to another embodiment of the present invention. In FIG. 12, an embodiment of adjusting a gain value in the ultrasound apparatus 100 in a method other than that described with reference to FIGS. 10A to 11 when a three-point input is received in the absolute mode is described.

First, a user input unit 1205 shown in the bottom of FIG. 12 is described. The ultrasound apparatus 100 receives a three-point input, which is a user input of selecting three or more locations on the user input unit 1205. For example, the ultrasound apparatus 100 may receive a three-point input of selecting three points in a subarea 1211 or four points in a subarea 1213 by a user.

Thereafter, when a user input of dragging three fingers from the three points in the subarea 1211 to a subarea 1212 is detected, the ultrasound apparatus 100 detects a location corresponding to a boundary in a dragging direction. That is, the ultrasound apparatus 100 may detect a location 1221, which corresponds to a boundary in the right direction, from among three points in the subarea 1212.

Likewise, when four fingers are dragged from four points in the subarea 1213 to a subarea 1214 in the left direction, the ultrasound apparatus 100 may detect a location 1222, which corresponds to a boundary in the left direction, from among four points in the subarea 1214. Next, output units 1200 and 1210 shown in the top of FIG. 12 are described. The ultrasound apparatus 100 may determine locations 1223 and 1224, which correspond to the locations 1221 and 1222, in a second region included in the output unit 1200 according to the detection of the locations 1221 and 1222, respectively. Thereafter, the ultrasound apparatus 100 may acquire gain values corresponding to the locations 1223 and 1224.

The ultrasound apparatus 100 may display a second gain line indicating gain values before being adjusted to the gain values corresponding to the locations 1223 and 1224 on a screen.

Finally, the ultrasound apparatus 100 may apply, to the ultrasound data, the gain values corresponding to the locations 1223 and 1224 and display a third gain line 1225 indicating the gain values applied to the ultrasound data on the output unit 1210.

As described above with reference to FIGS. 8 to 11, the user may adjust a gain value by concretely forming a first gain line in a user input unit. In addition, as in the embodiment described with reference to FIG. 12, the user may adjust a gain value by using a three-point input of dragging three or more fingers on three or more locations.

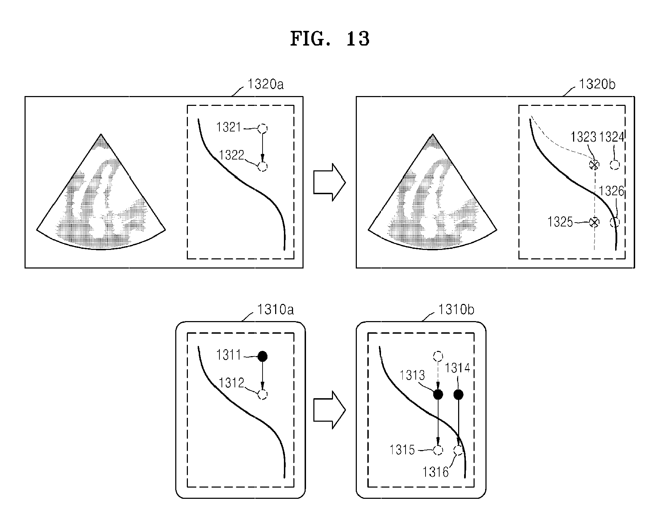

FIG. 13 illustrates a multi-input according to an embodiment of the present invention. The multi-input indicates an input in which a user input changes from a one-point input to a two-point input (or vice versa). An embodiment of a multi-input of changing from a one-point input to a two-point input is shown at the bottom of FIG. 13.

In FIG. 13, the one-point input and the two-point input are only an example of the multi-input. The multi-input may include not only a case where an order of the one-point input and the two-point input is changed but also a user input of a new form combined with various different types of user inputs.

First, a user input unit 1310a receives a user input (a one-point input) of dragging a finger from a location 1311 to a location 1312. Thereafter, a user input unit 1310b receives a user input (a two-point input) of dragging two fingers from locations 1313 and 1314 to respective locations 1315 and 1316. The one-point input and the two-point input of FIG. 13 may be continuously input. That is, a user drags a finger from the location 1311 to the location 1312 and further touches the location 1314 with another finger while maintaining the touch on the location 1312 (i.e., the location 1313). Thereafter, the user drags the two fingers from the locations 1313 and 1314 to the respective locations 1315 and 1316.

According to the embodiment of FIG. 13, the ultrasound apparatus 100 may not acquire a gain value of the location 1312 maintained from the one-point input when the user changes the one-point input to the two-point input. That is, the ultrasound apparatus 100 may display only a pointer indicating locations 1321 and 1322 on an output unit 1320a and may not acquire the gain value for the first received one-point input.

However, when the two-point input is received immediately after the one-point input, the ultrasound apparatus 100 may acquire a gain value of the location 1313, which is a location maintained from the one-point input, from among the locations 1313 and 1314 of the two-point input. Furthermore, the ultrasound apparatus 100 may acquire a gain value connecting a location 1323 to a location 1325, which respectively correspond to the location 1313 and the location 1315, and may adjust ultrasound data according to the acquired gain value.

As described above, the ultrasound apparatus 100 may display a third gain line indicating the gain value adjusted to correspond to the locations 1323 and 1325 as a dashed line on an output unit 1320b. Thereafter, when the user ends the two-point input at locations 1315 and 1316, the ultrasound apparatus 100 may display the third gain line as a solid line.

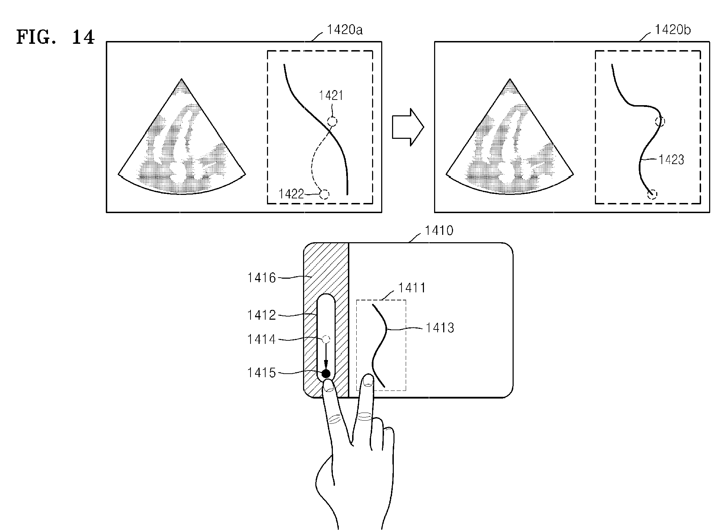

FIG. 14 illustrates a one-point input, which is input together with a detection signal, according to an embodiment of the present invention. The detection signal may be received through a third region 1412, which is a separate region from a first region 1411 of a user input unit 1410, and received together with the one-point input for forming a first gain line 1413.

The detection signal may be received as a physical or electrical signal through a partial region 1416 distinguished from a region for receiving a touch input in the user input unit 1410. That is, the ultrasound apparatus 100 may receive the detection signal through sensing parameters, such as a pressure, a current, a temperature, and the like.

When the detection signal of dragging a finger from a location 1414 to a location 1415 through the third region 1412 is received together with a user input for the first gain line 1413, the ultrasound apparatus 100 may detect the user input when received together with the detection signal. A location of the user input for forming the first gain line 1413 when the reception of the detection signal starts is a location 1421.

That is, if the detection signal starting from the location 1414 is received while a one-point input for forming the first gain line 1413 in the first region 1411 is being received from the user, the ultrasound apparatus 100 may not acquire a gain value for the one-point input until the detection signal is received.

In other words, only after the detection signal is received, the ultrasound apparatus 100 may acquire a gain value from the location 1421 to a location 1422 at which the detection signal is received and display a pointer on an output unit 1420a based on the user input for forming the first gain line 1413; otherwise, the user input for forming the first gain line 1413 is ignored.

Thereafter, the ultrasound apparatus 100 may apply the gain value from the location 1421 to a location 1422 to ultrasound data and display a third gain line 1423 indicating the adjusted gain value on an output unit 1420b.

According to the embodiment of FIG. 14, the user may selectively adjust a gain value in combination with the use of a detection signal.

The third region 1412 of FIG. 14 may be used as not only a means for receiving a detection signal as described above but also a physical guide for a touch input.

That is, the ultrasound apparatus 100 may use the third region 1412 as a physical reference for identifying the first region 1411 for detecting a user input in the user input unit 1410. The user may use the third region 1412 as a reference for identifying a vertical axis of the first region 1411 from the user input unit 1410. In other words, the user may guess a location of a user input for a vertical axis coordinate of the first region 1411 by touching the third region 1412 formed of a physical member distinguished from the partial region 1416, and the third region 1412 may aid in guiding the user's input with respect to the location of a vertical axis coordinate of the first region 1411. Accordingly, the user may overcome a physical limitation of a touch input when the user fixes his/her eyes on the output unit 1420a or 1420b.

In the current embodiment, the third region 1412 may be formed of a member distinguished from the other part of the user input unit 1410 or may be implemented in a protrusion or groove shape from the partial region 1416.

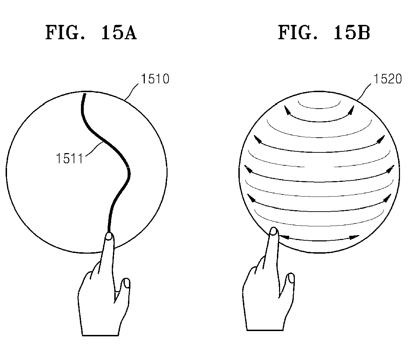

FIGS. 15A and 15B illustrate using a track ball according to embodiments of the present invention. FIGS. 15A and 15B show different embodiments using a track ball.

In FIG. 15A, the ultrasound apparatus 100 may detect a rotation direction and a rotation degree of a track ball that is a user input unit 1510 when a user input for forming a first gain line 1511 for the track ball is received from a user. Thereafter, the ultrasound apparatus 100 may adjust a gain value based on the detected rotation direction and rotation degree of the track ball.

That is, the ultrasound apparatus 100 may match a distance from the top to the bottom of the track ball with a depth axis of ultrasound data and adjust a gain value of the ultrasound data based on a rotation direction and a rotation degree varying along locations in a vertical direction of the track ball.

In FIG. 15B, the ultrasound apparatus 100 may divide a track ball that is a user input unit 1520 into a plurality of sections along locations in a vertical direction of the track ball and adjust a gain value of ultrasound data based on a user input in a left or right direction, which is received through each of the plurality of sections. Although FIG. 15B shows only six sections using five arrows, the user input unit 1520 may include a smaller or larger number of sections. Accordingly, the ultrasound apparatus 100 may match the plurality of sections with a depth axis of ultrasound data and adjust a corresponding gain value of the depth axis based on a user input received at each section.

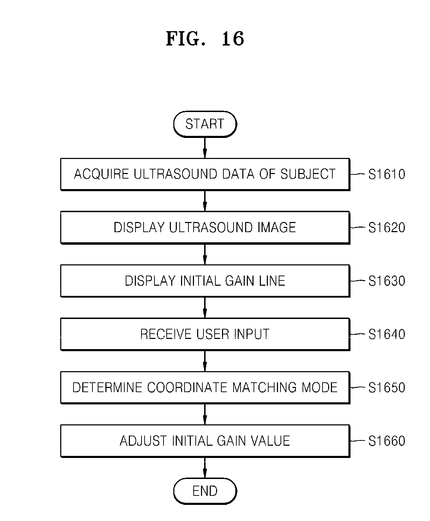

FIG. 16 is a flowchart illustrating a method of inputting ultrasound information, according to another embodiment of the present invention. Since operations S1610 and S1620 are the same as operations S310 and S330 of FIG. 3, a detailed description thereof is omitted.

In operation S1630, unlike the embodiment of FIG. 3, the ultrasound apparatus 100 displays an initial gain line indicating an initial gain value internally determined by a system. That is, the ultrasound apparatus 100 may internally determine the initial gain value determined according to a depth value of ultrasound data of an object as acquired in operation S1610. Accordingly, the ultrasound apparatus 100 may display the initial gain line indicating the initial gain value.

In operation S1640, the ultrasound apparatus 100 receives a user input for adjusting the initial gain line. That is, the ultrasound apparatus 100 may detect a touch input of a user through a first region included in a user input unit. The description with respect to FIGS. 5 to 15B may be applied to various types of a touch input of the user.

In operation S1650, the ultrasound apparatus 100 determines a coordinate matching mode in the same manner as operation S370 of FIG. 3. When the coordinate matching mode is the relative mode in operation S1650, the ultrasound apparatus 100 may match a location at which a user input starts with a location on the initial gain line displayed in operation S1630.

In operation S1660, the ultrasound apparatus 100 adjusts a gain value of the ultrasound data based on the user input received in operation S1640 and the coordinate matching mode determined in operation S1650. That is, the ultrasound apparatus 100 may adjust the internally determined initial gain value according to the ultrasound data by adjusting the initial gain value according to the user input.

Like the description with reference to FIG. 3, after operation S1660, the ultrasound apparatus 100 may display an ultrasound image in which the gain value adjusted from the initial gain value is reflected.

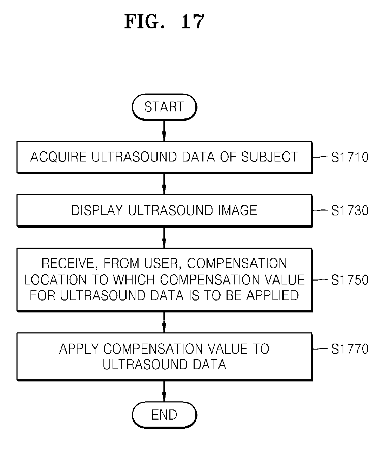

FIGS. 17 to 18C illustrate a quick scan for simply and efficiently adjusting a compensation value of ultrasound data according to an embodiment of the present invention. FIG. 17 is a flowchart illustrating a method of inputting ultrasound information, according to another embodiment of the present invention.

In operations 1710 and 1730, the ultrasound apparatus 100 acquires ultrasound data and displays an ultrasound image based on the ultrasound data. Since operations 1710 and 1730 are the same as the description with reference to FIG. 3, a detailed description thereof is omitted.

In operation S1750, the ultrasound apparatus 100 receives a compensation location, selected by a user, to which a compensation value for the ultrasound data is to be applied. The compensation value includes various types of image information applicable to the ultrasound data, for example, brightness, chroma, color, definition, and the like.

In operation S1770, the ultrasound apparatus 100 applies the compensation value to the ultrasound data at the location received in operation S1750. That is, the ultrasound apparatus 100 may perform an automatic compensation process for the location selected by the user.

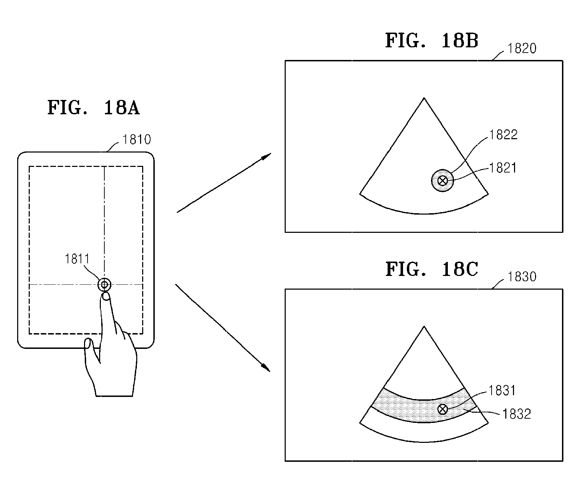

FIGS. 18A to 18C illustrate the automatic compensation process according to embodiments of the present invention.

In FIG. 18A, the ultrasound apparatus 100 receives a user input for selecting a location 1811 through a user input unit 1810. A vertical axis of a first region included in the user input unit 1810 matches a depth axis of an ultrasound image displayed through an output unit 1820 or 1830.

In FIG. 18B, when the location 1811 of FIG. 18A is selected, the ultrasound apparatus 100 may detect a location 1821 matching the location 1811. Thereafter, the ultrasound apparatus 100 applies a compensation value to ultrasound data corresponding to the location 1821 and a surrounding region 1822. The surrounding region 1822 may be a region of interest (ROI) of a predetermined size.

According to the embodiment of FIG. 18B, the ultrasound apparatus 100 may select a compensation location based on both a horizontal axis and a vertical axis of a user input inputted into the user input unit 1810.

In FIG. 18C, the ultrasound apparatus 100 may detect a location 1831 matching the location 1811. Thereafter, the ultrasound apparatus 100 may apply the compensation value to not only an area around the location 1831 but also to corresponding ultrasound data on a same depth axis as the location 1831.

According to the embodiment of FIG. 18C, the ultrasound apparatus 100 may select a compensation location by only considering a vertical axis coordinate of a user input inputted into the user input unit 1810. That is, the ultrasound apparatus 100 may compensate ultrasound data with the same value as that of a user input of the same vertical axis coordinate regardless of a horizontal axis coordinate.

According to the ultrasound information input method and the ultrasound apparatus described above, a user may adjust a gain value of ultrasound data while maintaining his/her visual focus on an ultrasound image. According to the embodiments described above, the user may simply and efficiently adjust a gain value.

In addition, by allowing the user to maintain operation experiences of the user on a hardware device and simultaneously enable a quick operation of the hardware device, a time taken to diagnose an object may be reduced.

The methods of the present invention can be written as computer programs and can be implemented in general-use digital computers that execute the programs using a computer-readable recording medium. In addition, a structure of data used in the methods can be recorded in a computer-readable recording medium in various ways. It should be understood that program storage devices, which may be used to describe a storage device including executable computer codes for executing the methods of the present invention, do not include temporary objects, such as carrier waves and signals. Examples of the computer-readable recording medium include storage media such as magnetic storage media (e.g., ROM, floppy disks, hard disks, etc.) and optical recording media (e.g., CD-ROMs, or DVDs).

It will be understood by those of ordinary skill in the art that various changes in form and details may be made therein without departing from the spirit and scope of the present invention as defined by the following claims. The exemplary embodiments should be considered in a descriptive sense only and not for purposes of limitation. Therefore, the scope of the present invention is defined not by the detailed description of the present invention but by the appended claims, and all differences within the scope will be construed as being included in the present invention.

## Claims

1. A method of inputting ultrasound information in an ultrasound apparatus comprising a user input unit for receiving a touch input, the method comprising:
displaying an ultrasound image based on ultrasound data acquired from an object;
receiving, from a user, through a first region included in the user input unit, a first gain line for setting a gain value to be applied to the ultrasound data;
determining a coordinate matching mode between a second region included in a screen on which the ultrasound image is displayed and the first region; and
adjusting a gain value of the ultrasound data based on the coordinate matching mode and the first gain line.

2. The method of claim 1, wherein the gain value of the ultrasound data includes at least one of a time gain compensation (TGC) value, a lateral gain compensation (LGC) value, an overall gain value, and a partial gain value.

3. The method of claim 1, wherein the receiving comprises:
detecting a user input of tapping and/or dragging one or more locations in the first region; and
acquiring a gain value corresponding to the user input.

4. The method of claim 3, wherein the coordinate matching mode is a relative mode in which a vertical axis coordinate of the first region matches a depth axis coordinate of the second region or an absolute mode in which a horizontal axis coordinate and the vertical axis coordinate of the first region match a horizontal axis coordinate and the depth axis coordinate of the second region.

5. The method of claim 4, wherein when the coordinate matching mode is the relative mode, the horizontal axis coordinate of the first region, from which the user input starts, matches a predetermined point on a horizontal axis of the second region.

6. The method of claim 5, wherein the predetermined point is located on a second gain line corresponding to the gain value of the ultrasound data before the adjusting.

7. The method of claim 4, wherein when the coordinate matching mode is the absolute mode, coordinates of the first region, from which the user input starts, are coordinates of the second region matching coordinates of the first region.

8. The method of claim 1, wherein the user input unit comprises at least one of a touch screen, a touch panel, a touch pad, and a track ball.

9. The method of claim 1, further comprising displaying on a screen an ultrasound image based on the ultrasound data having the adjusted gain value.

10. The method of claim 1, further comprising displaying on a screen at least one of a second gain line corresponding to the gain value of the ultrasound data before the adjusting and a third gain line corresponding to the adjusted gain value.

11. The method of claim 1, wherein the first gain line is information regarding the touch input received by the user input unit.

12. The method of claim 3, wherein the detecting comprises:
determining a detection region of a predetermined size, which includes a location at which the user input starts; and
determining a region except for the detection region as a non-detection region in which the touch input is not detected.

13. The method of claim 12, further comprising clearing the detection region and the non-detection region after a predetermined time has elapsed from the end of the user input.

14. An ultrasound apparatus comprising:
an acquisition unit for acquiring ultrasound data from an object;
an output unit for displaying an ultrasound image based on the ultrasound data;
a user input unit, which includes a first region and receives, from a user and through the first region, a first gain line for setting a gain value to be applied to the ultrasound data;
a mode checking unit for determining a coordinate matching mode between a second region included in the output unit and the first region; and
an image processing unit for adjusting a gain value of the ultrasound data based on the coordinate matching mode and the first gain line.

15. A non-transitory computer-readable storage medium having stored therein program instructions, which when executed by a computer, perform the method of claim 1.
